# EUROPEAN PATENT APPLICATION

(11) **EP 0 548 559 A1**
(43) Date of publication of application: **30.06.1993**
(21) Application number: 92119892.5
(22) Date of filing: 23.11.1992
(51) Int. Cl.: C07D 213/80, C07D 213/79

(54) **Process for the preparation of dialkyl pyridine -2,3-dicarboxylates and derivatives thereof from dialkyl dichloromaleate**

(30) Priority: 27.12.1991 US 815315
(71) Applicant: AMERICAN CYANAMID COMPANY, Wayne, NJ 07470-8426 (US)
(72) Inventor: Chiarello, George Anello, Yardville, New Jersey 08620 (US)
(74) Representative: Wächtershäuser, Günter, Prof. Dr.

(57) **Abstract**

There is provided a process for the preparation of substituted and unsubstituted-2,3-pyridinedicarboxylate compounds by reacting a dialkyl dichloromaleate with a dialkylamine to form an enamine intermediate and reacting said intermediate with an appropriately substituted α,β-unsaturated aldehyde or ketone and an ammonia source, as shown in Flow Diagam I:

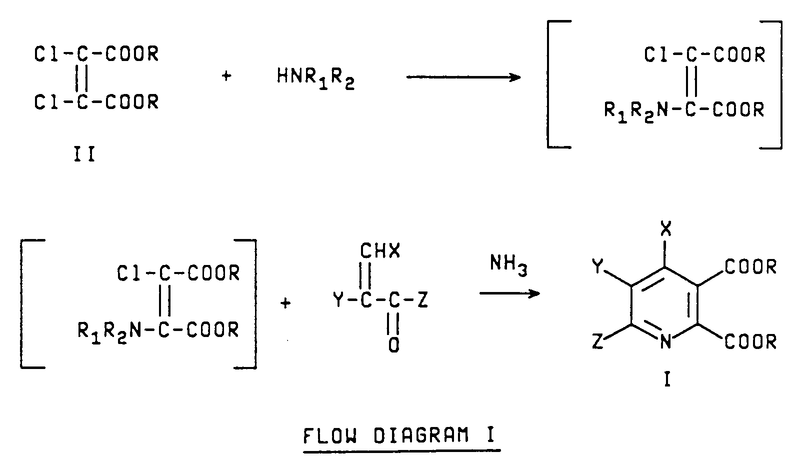

## Description

Pyridine-2,3-dicarboxylates are useful intermediates in the preparation of highly effective herbicidal 2-(2-imidazolin-2-yl)nicotinic acids, esters and salts. Imidazolinyl nicotinates and derivatives thereof are highly effective herbicides at low rates of application and demonstrate selective control of noxious weeds in the presence of important agronomic crops while exhibiting exceptionally low mammalian toxicity.

It is an object of this invention to provide a method for the preparation of substituted and unsubstituted-2,3-pyridinedicarboxylates utilizing dialkyl dichloromaleate, a dialkylamine, an appropriately substituted-α,β-unsaturated aldehyde or ketone and an ammonia source.

The invention herein described relates to a novel method for the preparation of substituted and unsubstituted 2,3-pyridinecarboxylates of formula I 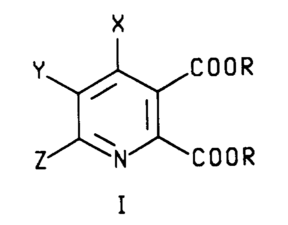
wherein R is C₁-C₆alkyl; X is hydrogen, halogen, C₁-C₄alkyl, C₁-C₄alkoxy or C₂-C₅alkenyl, Y is hydrogen, halogen, C₁-C₆alkyl optionally substituted with one to three halogens, hydroxy groups, C₁-C₄alkoxy groups or C₁-C₄-alkylthio groups, C₁-C₄alkoxycarbonyl, aminocarbonyl, phenyl, substituted phenyl, phenoxy, substituted phenoxy, phenylthio or substituted phenylthio and Z is hydrogen, C₁-C₆alkyl or C₂-C₆alkenyl.

Compounds of formula I are useful as intermediates in the preparation of herbicidal 2-(2-imidazolin-2-yl)nicotinates as illustrated below. 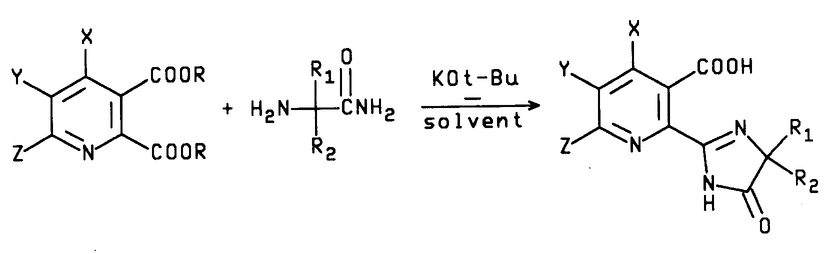
wherein R₁ is hydrogen or C₁-C₄alkyl; R₂ is hydrogen, halogen, C₁-C₄alkyl, C₃-C₆cycloalkyl or R₁ and R₂ may be taken together to form a C₃-C₆cycloalkyl optionally substituted with methyl and X, Y and Z are as described for formula I.

It has now been found that pyridine-2,3-dicarboxylates of formula I may be prepared from dialkyl dichloromaleates of formula II by reacting said maleate with a dialkylamine in the presence of a suitable solvent, optionally at an elevated temperature to form an enamine intermediate and reacting said intermediate with at least one molar equivalent of an α,β-unsaturated aldehyde or ketone of formula III wherein X, Y and Z are as described above for formula I in the presence of an acid and an ammonia source. The processs is shown in Flow Diagram I. 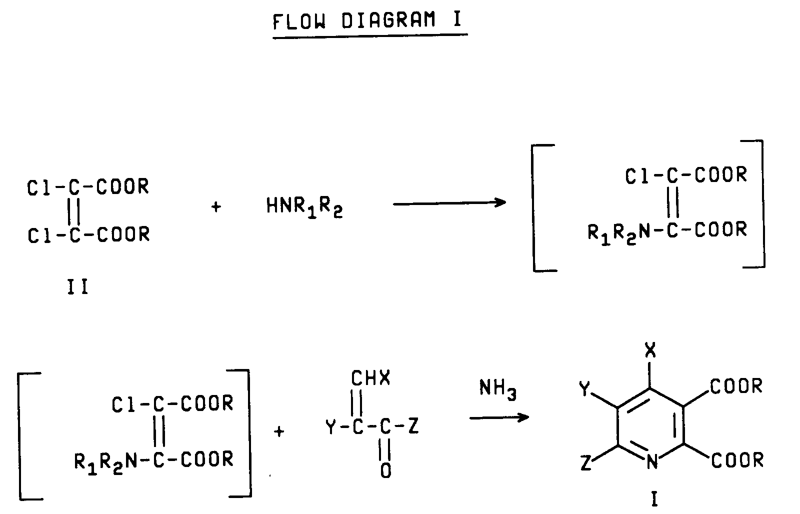
Dialkylamines, HNR₁R₂, wherein R₁ and R₂ are each independently C₁-C₆alkyl such as dimethylamine, diethylamine, diisopropylamine, dipropylamine, dibutylamine, methyl-n-butylamine, ethylisopropylamine and the like are suitable for use in the process of the invention.

Among the solvents suitable for use in the inventive process are polar solvents such as alcohols, nitriles such as acetonitrile, carboxylic acid amides such as N,N-dimethylformamide, N-methylpyrrolidone, sulfoxides such as dimethylsulfoxide, sulfones and the like.

Among the α,β-unsaturated aldehydes or ketones of formula III which may be used in the process of the invention are acrolein, methacrolein, ethacrolein, crotonaldehyde, methyl vinyl ketone, α-n-butylacrolein, 4-methyl-2-hexanal, α-methoxymethacrolein, α-chloromethacrolein, α-trifluoromethacrolein, cinnamaldehyde, α-ethoxyacrolein, methyl α-formylacrylate, α-(2-cyanoethyl)acrolein and the like.

Ammonia sources may include organic ammonium salts such as ammonium acetate, ammonium propionate and the like, inorganic ammonium salts such as ammonium sulfate, ammonium carbonate, ammonium carbamate, ammonium sulfamate and the like, quaternary ammonium salts such as tetraalkylammonium halides, tetraalkylammonium sulfates and the like. Of course, ammonia itself may also be used as the ammonia source in the process of the invention.

Thus, pyridine-2,3-dicarboxylates containing substituents in the 4, 5 and 6 positions may be conveniently prepared by admixing a formula II dialkyl dichloromaleate with a dialkylamine in a suitable solvent, optionally heating said mixture until the reaction is essentially complete, optionally filtering said reaction mixture, optionally adding to the filtrate or to the unfiltered reaction mixture an acid such as a mineral acid, sulfuric acid, phosphoric acid, an organic acid such as acetic acid or propionic acid and the like, preferably acetic acid, and adding at least one molar equivalent of an α,β-unsaturated aldehyde or ketone of formula III and an ammonia source, optionally heating the resulting reaction mixture until formation of the formula I pyridinedicarboxylate is complete. The reaction product may be isolated using standard purification techniques such as fractional distillation, recrystallization, extraction, liquid chromatography and the like.

The rate of formation of the engine intermediate and the formula I pyridinedicarboxylate is temperature dependent, thus, reaction time may be diminished by heating the reaction mixtures at temperatures of about 45^{o}C or greater.

In one embodiment of the invention, the enamine intermediate may be prepared and isolated prior to reaction with the α,β-unsaturated aldehyde or ketone of formula III and subsequently reacted with the formula III compound and an ammonia source to give a pyridine-2,3-dicarboxylate of formula I. For example, dialkyl chlorooxalacetate may be reacted with at least one molar equivalent of a dialkylamine in the presence of a polar solvent at an elevated temperature to give the desired enamine compound of formula IV. The reaction is shown in flow diagram II 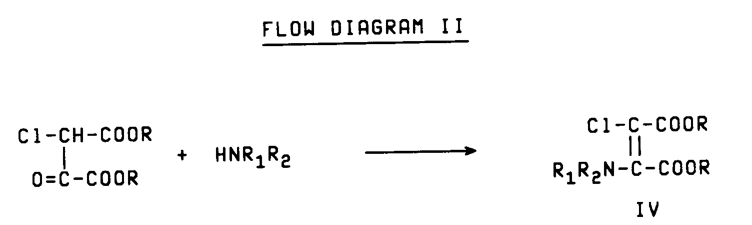
wherein R, R₁ and R₂ are each independently C₁-C₆alkyl.

The enamine compound of formula IV may be reacted with at least one molar equivalent of an α,β-unsaturated aldehyde or ketone of formula III in the presence of a solvent and an ammonia source, optionally in the presence of an acid, to give the desired pyridinedicarboxylate of formula I. The reaction is illustrated in flow diagram III. 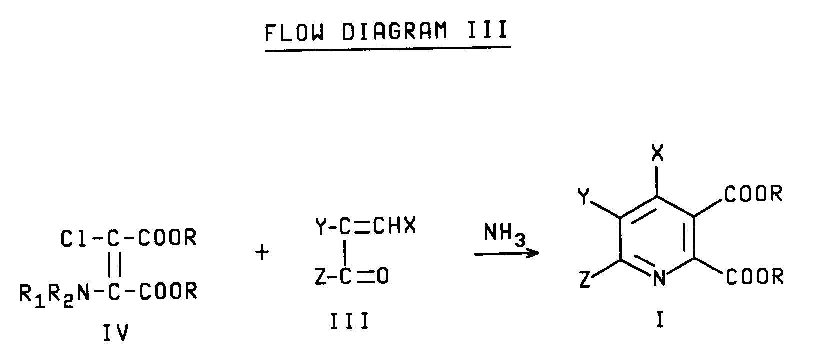
Solvents suitable for use are as described hereinabove for flow diagram I. Reaction time may be diminished by heating the reaction mixture at temperatures of about 45^{o}C or greater.

In order to facilitate a further understanding of the invention, the following examples are presented primarily for the purpose of illustrating certain more specific details thereof and the invention is not to be deemed limited thereby. The term HPLC designates high performance liquid chromatography. Unless otherwise noted, all parts are parts by weight.

### EXAMPLE 1

### Preparation of diethyl 5-ethylpyridine-2,3-dicarboxylate

A stirred mixture of diethyl dichloromaleate (4.8g, 0.02 mole) in toluene is treated with dipropylamine (2.23g, 0.02 mole), heated at 60^{o}-65^{o}C for 4 hours, then heated at 90^{o}C for 4 additional hours, cooled to room temperature and concentrated in vacuo. The concentrate is diluted with methylene chloride, treated with 2-ethylacrolein (1.8g, 0.02 mole), further treated with ammonium acetate (2.3g, 0.03 mole) and absolute ethanol, stirred at ambient temperatures for 48 hours and concentrated in vacuo to yield the title product, identified by HPLC analysis.

### EXAMPLE 2

### Preparation of dialkyl 5-substituted pyridine-2,3-dicarboxylates

Using essentially the same procedure described in Example 1 and substituting the appropriate acrolein derivative, the following corresponding pyridine-2,3-dicarboxylates are obtained.

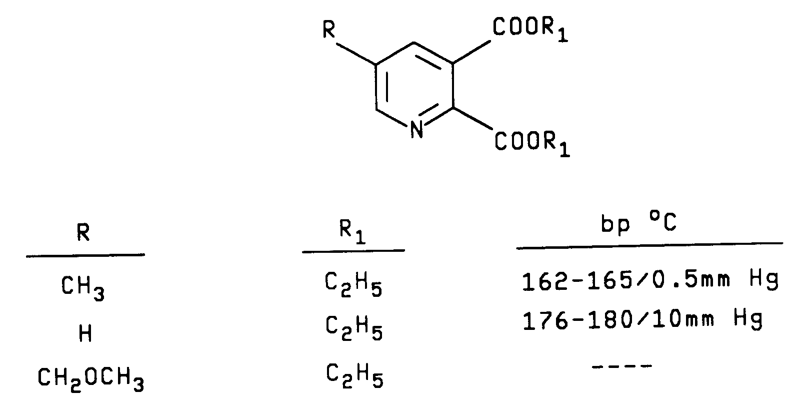

## Claims

1. A process for the preparation of a compound of formula I 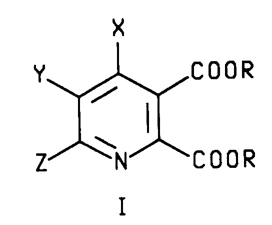 wherein R is C₁-C₆alkyl; X is hydrogen, halogen, C₁-C₄alkyl, C₁-C₄alkoxy or C₂-C₅alkenyl; Y is hydrogen, halogen, C₁-C₆alkyl optionally substituted with one to three halogens, hydroxy groups, C₁-C₄alkoxy groups or C₁-C₄alkylthio groups, C₁-C₄alkoxycarbonyl, aminocarbonyl, phenylthio, substituted phenylthio, phenoxy, substituted phenoxy, phenyl or substituted phenyl and Z is hydrogen, C₁-C₆alkyl or C₂-C₆alkenyl, characterized by reacting a dialkyl dichloromaleate of formula II 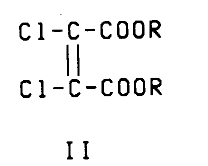 wherein R is C₁-C₆alkyl with a dialkylamine, HNR₁R₂ wherein R₁ and R₂ are each independently C₁-C₆alkyl, in the presence of a solvent to form an enamine intermediate, optionally filtering off insolubles, and reacting said intermediate with at least one molar equivalent of an α,β-unsaturated aldehyde or ketone of formula III 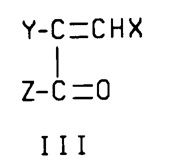 wherein X, Y and Z are as described above, an ammonia source and optionally an acid in the presence of the solvent to form the pyridinedicarboxylate compound of formula I.

2. A process for the preparation of a compound of formula I 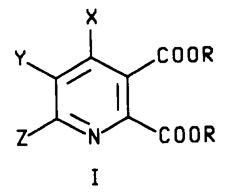 wherein R is C₁-C₆alkyl; X is hydrogen, halogen, C₁-C₄alkyl, C₁-C₄alkoxy or C₂-C₅alkenyl; Y is hydrogen, halogen, C₁-C₆alkyl optionally substituted with one to three halogen, hydroxy, C₁-C₄alkoxy or C₁-C₄alkylthio groups, C₁-C₄alkoxycarbonyl, aminocarbonyl, phenylthio, substituted phenylthio, phenoxy, substituted phenoxy, phenyl or substituted phenyl and Z is hydrogen, C₁-C₆ alkyl or C₂-C₆alkenyl characterized by reacting a dialkyl aminochlorobutenedioate of formula IV 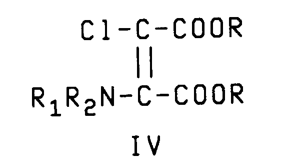 wherein R, R₁ and R₂ are each independently C₁-C₆ alkyl with at least one molar equivalent of an α,β-unsaturated aldehyde or ketone of formula III 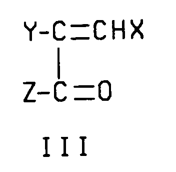 wherein X, Y and Z are as described above in the presence of a solvent, an ammonia source and optionally in the presence of an acid.

3. The process according to claim 1 or claim 2 for the preparation of a compound of formula I wherein X and Z are hydrogen and Y is hydrogen, halogen or C₁-C₆alkyl optionally substituted with one to three halogens, C₁-C₄alkoxy groups or C₁-C₄alkylthio groups.

4. The process according to claim 1 wherein the solvent is a polar solvent, the dialkylamine is dipropylamine, and the reaction is conducted at a temperature of about 45^{o}C or greater.

5. The process according to claim 2 wherein the temperature is about 45^{o}C or greater.

6. The process according to claim 3 wherein the formula I compound is selected from the group consisting of dialkyl pyridine-2,3-dicarboxylate, dialkyl 5-methyl-2,3-pyridinedicarboxylate, dialkyl 5-ethyl-2,3-pyridinedicarboxylate, dialkyl 5-(methoxymethyl)-2,3-pyridinedicarboxylate, or dialkyl 5-(chloromethyl)-2,3-pyridinedicarboxylate.
